# EUROPEAN PATENT APPLICATION

(11) **EP 2 214 048 A1**
(43) Date of publication of application: **04.08.2010**
(21) Application number: 07858262.4
(22) Date of filing: 27.11.2007
(51) Int. Cl.: G02C 7/10, A61F 9/00

(54) **GLASSES FOR SAFETY AND PREVENTION, COMPRISING A TREATED SURFACE, FOR THE PROTECTION AND TREATMENT OF EYES IN CERTAIN OCCUPATIONS AND DURING SPORT**

(30) Priority: 26.10.2007 ES 200702837
(71) Applicant: Universidad Complutense de Madrid, 28040 Madrid (ES)
(72) Inventor: SANCHEZ RAMOS, Celia, E-28040 Madrid (ES)
(74) Representative: Temino Ceniceros, Ignacio
(86) International application number: PCT/ES2007/000685
(87) International publication number: WO 2009/053502

(57) **Abstract**

The invention relates to a transparent filtering element for preventing damage to healthy eyes and for the treatment and prophylaxis of eyes which are pseudo-aphakic and/or show macular or retinal degeneration. The invention is **characterised in that** the element is produced by applying a filter with a yellow pigmentation onto the transparent or translucid surface(s) of protective glasses, in order to protect the eyes from the short wavelengths of the visible spectrum (between 500 and 380 nm). The invention can be used, for example, in occupations wherein the person is exposed to high light intensities (fishermen, welders) or for sport (scuba diving, tennis, ski...). The invention is compatible with other treatments.

## Description

### OBJECT OF THE INVENTION

The invention is part of the ophthalmology sector, within optic applications of a preventive and therapeutic nature

The purpose of this invention are glasses for the protection of healthy eyes, pseudo-aphakic (operated from cataracts) and/or macular and retinal degeneration, against short wavelengths, which is accomplished by the application of a filter with yellow pigmentation over the transparent or translucid surface, or surfaces of common protective glasses, for example, a screen or face shield type, for the purpose of protecting the eyes from short wavelengths of the visible spectrum (from 500 to 380 nm). As an example, the application of this invention is to be used while performing jobs exposed to high intensity lighting (fishermen, welders, etc.) or sports (scuba diving, tennis, ski, etc.). This invention is compatible with other treatments.

### STATE OF THE ART

The perception of vision is the result of the response to visible radiation from 380 to 760 nm. In the environment, solar radiation is the main hazard to vision. The sun emits UV and IR radiations, which are almost entirely absorbed by the atmosphere. The solar radiation that is transmitted through the atmosphere, when it reaches the earth's surface, consists in UV-B rays (from 230 to 300 nm), UV or UV-A rays (from 300 to 380 nm), visible light (from 380 to 760) and IR rays (from 760 to 1400 nm). Human eyes that are perfectly healthy freely transmit the IR rays and the majority of the visible spectrum to the retina but the cornea and the crystalline prevent the most reactive waves of the visible spectrum (the UV-B rays and the blue light portion of the visible spectrum) from reaching the retina.

However, the human crystalline changes its transmission characteristics as it ages, intensifying its yellow colour and increasing its capability for filtering UV and blue light rays. For this reason, the violet light (<400nm) is not transmitted in people older than 65 and the transmission of blue light (from 400 to 500 nm) is significantly reduced.

On the other hand, the retina auto-protects itself from the short wavelengths in two ways: with a heterogeneous distribution of the photo-receptors in such a way that photo-receptors, which are sensitive to blue light, do not exist in the macular depression and by the action of existing yellow pigments in the same area, which also perform a protective function.

These natural protections of the human eye against shorter wavelengths - the crystalline and the retinas themselves - can be seriously affected by certain pathologies and/or surgical procedures:
- Cataracts, which can only be treated surgically and entails the extraction of the crystalline.
- A pathological aging process occurs frequently and produces a degradation of the retina structures producing the macular degeneration associated with age (MDAA).

Historically, we must consider the convergence in the same demographic group - persons older than 65 years - of these two pathologies: the cataract and the MDAA. Cataracts are the main cause of loss of vision and the MDAA of blindness in this same demographic group. Additionally, we must consider the presumable increment of both pathologies; among other things due to the increase in life expectancy and, therefore, these provoke great interest in the areas of research and their application in the industry.

Therefore, as explained in the scientific bibliography, several epidemiological studies have been evaluated by the cataract surgery association and the macular degeneration associated with aging (MDAA). The works by Klein (Klein R, Klein BE, Wong TY, Tomany SC, Cruickshanks KJ. The association of cataract and cataract surgery with the long-term incident of age-related maculopathy. Arch Ophthalmol 120:1551-1558.2002) and Freeman (Freeman E, Munoz B, West SK, Tielsch JM, Schein OD. Is there an association between cataract surgery and age-related macular degeneration. Am J Ophthalmolm 135(6): 849-856.2003) assure the existence of a higher risk of developing the MDAA symptoms in people who have had cataract surgery. However, the prior investigations by Wang (Wang JJ, Mitchell P, Cumming RG, Lim R. Cataract and age-related maculopathy: the Blue Mountains Eye Study. Ophthalmic Epidemiol 6: 317-326.1999) and McCarty (McCarty CA, Mukesh BN, Fu CL, Mitchell P, Wang JJ, Taylor HR. Risks factors for age-related maculopathy: the Visual Impairment Project. Arch Ophthalmol 119:1455-1462.2001) reject this hypothesis, possibly due to a less developed level of applied technology for diagnostic measurement. It is only recent the implementation of techniques, like Optic coherent Tomography, that allow a follow up of the evolution of the nuero-degenerative retina processes in a rigorous, immediate and non-invasive way.

This is an important fact for knowing the determining effect of the natural pigments that absorb harmful radiations.

On the other hand, some techniques have been developed to protect eyes that have been operated from cataracts from short wavelengths:
- Several types of filters exist in the market which use a yellow pigmentation, but there still isn't an optimum procedure and/or device to apply these filters to the human eyes as a therapeutic and preventive measure to substitute and/or improve their natural protection.
- Since mid 90's, intraocular lenses provided with a yellow filter have been implanted on eyes operated from cataracts. This alternative involves a surgical procedure with all its obvious risks and difficulties. Also, there exist a large number of people operated from cataracts to which a transparent intraocular lens has been implanted to replace the crystalline that does not have the necessary yellow pigmentation protection. In these cases, it is necessary to complement the artificial crystalline, which is exempt of yellow pigmentation, with the insertion of a yellow pigmentation support system; for example, the protective glasses purpose of this patent application.

Several patents related to this technique have also been developed, which present significant differences when compared to this invention. These patents may be categorized in three groups:
First of all, documents which refer to the protection against lasers:
   - Ocular protection devices against lasers that include an organic polymeric material cell (US5116113)
   - Goggle type glasses for protecting against lasers and other optic radiations (US3791721)
   - Ocular equipment against laser light for ballistics and light protection (US2005264753)
   - Ocular protection with wavelength modulation for protecting against military use lasers (US6128123)
   - Device for protecting and viewing the laser beam for use in glasses and protective covers (EP0240385)

These inventions differ from the one described herein in that they are designed for protecting against laser light and not against short wavelengths in the visible spectrum (from 500 to 380 nm).

Documents that refer to the application of any element that absorbs UV light over a protective element for the eyes, for use by operators or persons engaged in sports:
- Device for protecting against radiation for UV ray booths (EP1488768)
- Glasses to protect against glare for sport professionals (FR2846433)
- Glasses to protect against high sensitivity for intense lighting (CN2323387Y)
- Special protective glasses for drivers (GR 1002422)
- Safety glasses that adapt to the flow of light (RO113189)
- Safety glasses against hazardous light rays (EP0588384)
- Improvements in glasses for protecting the eyes against light for pilots exposed to extreme changes in the intensity of the light (GB483697)
- Glasses adapted for protection against blinding light (GB403067)
- Device for protecting vision when welding (EP1821137)
- Ultraviolet protective glasses for sun exposure, which have two filters joined by a transparent bridge (DE20200401143U)
- Colour night vision glasses that incorporate an optoelectronic filter and capable of protecting against lasers (US5756989)
- Protection apparatus to be used in night vision devices (US5751380)
- Eye protection device to protect operators against high radiation (GB1430183)
- Method for ocular protection (EP0115656) that includes a part that acts as a light attenuator and protects against UV and IR rays when welding and while performing other jobs.
- Protective screen adapted to welding goggles to protect the eyes against UV radiation (GB 559865)

None of these patents has as objective to protect or treat the eyes and none is carried out by incorporating the filtering material on to the physical surface of the eye.

Finally, documents that refer to the use of a yellow filter over other surfaces or on other devices for the protection and prophylaxis of the eyes:
- Therapeutic contact lens for pseudo-aphakic eyes and/or in the process of neuro-degeneration (US2006/0250)
- Therapeutic ophthalmic and prophylactic lens for pseudoaphakic eyes and/or in the process of neuro-degeneration (US2006/874)
- Illumination device with therapeutic and prophylactic filter for eyes that are healthy, pseudo-aphakic and/or in a neuro-degeneration process (US2006/0752)
- Prevention components for healthy eyes and treatment and prophylaxis for pseudo-aphakic eyes and/or in the process of neuro-degeneration for use in vehicles (US2006/0876)

The novelty offered by this invention, in relation with this last group of patents, stems from the fact that it materializes in an individual protection device in the shape of glasses (thus incorporating a shield or structure that supports the treated surfaces).

### DESCRIPTION OF THE INVENTION

In general, the purpose of the invention is the prevention and protection of the eyes against the absorption of blue and violet light using a filter applied, in the adequate proportion, to transparent or translucid surfaces of protective glasses -for example, of the screen or shield type. As mentioned above, it is particularly useful for persons with pseudo-aphakic eyes for functionally compensating for the extraction of protective pigments (removed during surgery) and in the case of neuro-degenerative processes for enhancing the prophylactic effect (processes that also, very frequently coincide in the same population group, the elderly), but it is equally important for the protection of any person's healthy eyes.

For this purpose, the invention is obtained as a result of the application on the transparent or translucid surface(s) of common protective glasses with a yellow pigmentation filter that absorbs short wavelengths from 500 to 380 nm. As an example, the application of this element in protective glasses for its use in occupations exposed to high intensity lighting (fishermen, welders, etc.) or sports (scuba diving, tennis, ski, etc.)

Therefore, the element combines two components:
- Protective glasses that include transparent or translucid surfaces
- The application of a filter with yellow pigmentation of those available on the market, compatible with the material on the surface, that absorbs short wavelengths from 500 to 380 nm, in the entire light transmission surface area.

### EMBODIMENT OF THE INVENTION

There exist several methods for applying this invention depending on the specific material of the surface where the filter is going to be applied. Additionally, the application method for this invention is illustrated in the following example, which is not limiting in scope since alternative combinations and ways for manufacturing this element exist:
Invention manufacturing example:
   - 10.3 mg of a conventional yellow pigment is dissolved, 4-Phenylazophenol, Solvent Yellow 7 (SY7), in 10.01 g of a solution of monomer containing 66% of PEA, 30.5% of PEMA and 3.3% of BDDA, resulting in a concentration of SY7 of 0.103 wt%
   - Subsequently, 52.3 mg of bis(4-tert-butylcyclohexyl) peroxydicarbonate as a polimerization catalyst.
   - Using a syringe, the solution is inserted in a mould shaped like the 2.5mm glasses. The solution is spread in 2.5 mm laminates. The mould may or may not include the rods that could be moulded afterwards and incorporated along with the corresponding accessories.
   - The polymerization is produced when inserting the mould in an oven at 65°C for 17 hours. The oven temperature is increased afterwards to 100°C for 3 additional hours.
   - Once the polymerization is finalized, the laminate is extracted from the mould, the appropriate checks for measuring the protection are carried out and it is subjected to its final carving.

In conclusion, the combination of the transparent or translucid surfaces of protection goggles and a yellow filter will provide protection of healthy eyes for any person in short wavelengths; in patients that have undergone cataracts surgery with intraocular transparent lens, correct the lack of protection of the operated eye, and eyes with neurodegenerative processes, improve and increase the natural protection of the eyes. In this way, the problems associated with alternative techniques that exist on the market (filters without application devices and intraocular lenses) are avoided.

## Claims

1. Element for protecting healthy eyes against short wavelengths **characterized in that** it is a result of combining the application of a yellow pigment filter that absorbs short wavelengths from 500 to 380 nm over the transparent or translucid surfaces of protective glasses, especially indicated for performing jobs or sports that expose persons to a high level of bright light.

2. Element for protecting healthy eyes against short wavelengths in accordance with claim 1 that includes a filter with yellow pigmentation suitable to be used on the transparent or translucid surface.

3. Element for the protection of healthy eyes against short wavelengths in accordance with claims 1 and 2 that includes one or several transparent or translucid surfaces of protective glasses.

4. Element for protecting the eyes against short wavelengths in accordance with claims 1, 2 and 3 **characterized**, for example, for being the result of applying a filter with yellow pigmentation that absorbs short wavelengths from 500 to 380 nm in the protective glasses.

5. Element in accordance with claims 1, 2, 3 and 4 **characterized** for filtering and being transparent.

6. Element in accordance with claims 1, 2, 3, 4 and 5 whose application is carried out on mask type protective glasses.

7. Element in accordance with claims 1, 2, 3, 4 and 5 whose application is carried out on shield type protective glasses.

8. Therapeutic and prophylactic element for pseudo-aphakic eyes **characterized** because it is a result of combining the application of a yellow pigment filter that absorbs short wavelengths from 500 to 380 nm over the transparent or translucid surfaces of protective glasses, especially indicated for performing jobs or sports that expose persons to a high level of bright light.

9. Therapeutic and prophylactic element for pseudo-aphakic eyes as per claim 8 that includes a filter with yellow pigmentation that is suitable to be used on the transparent or translucid surface.

10. Therapeutic and prophylactic element for the protection of pseudo-aphakic eyes in accordance with claims 8 and 9 that includes one or several transparent or translucid surfaces of protective glasses.

11. Therapeutic and prophylactic element for pseudo-aphalcic eyes in accordance with claims 8, 9 and 10 **characterized**, for example, for being the result of applying a filter with yellow pigmentation that absorbs short wavelengths from 500 to 380 nm in the protective glasses.

12. Element in accordance with claims 8, 9.10 and 11 **characterized** for acting as a filter and being transparent.

13. Element in accordance with claims 8, 9, 10, 11 and 12 whose application is carried out on mask type protective glasses.

14. Element in accordance with claims 8, 9, 10, 11 and 12 whose application is carried out on shield type protective glasses.

15. Therapeutic and prophylactic element for eyes with neurodegenerative retina process **characterized** because it is a result of combining the application of a yellow pigment filter that absorbs short wavelengths from 500 to 380 nm over the transparent or translucid surfaces of protective glasses, especially indicated for performing jobs or sports that expose persons to a high level of bright light.

16. Therapeutic and prophylactic element for eyes with neurodegenerative retina process as per claim 15 that includes a filter with yellow pigmentation that is suitable to be used on the transparent or translucid surface.

17. Therapeutic and prophylactic element for eyes with neurodegenerative retina process in accordance with claims 15 and 16 that includes one or several transparent or translucid surfaces of protective glasses.

18. Therapeutic and prophylactic element for eyes with neurodegenerative retina process in accordance with claims 15, 16 and 17 **characterized**, for example, for being the result of applying a filter with yellow pigmentation, that absorbs short wavelengths from 500 to 380 nm, of protective glasses.

19. Element in accordance with claims 15, 16, 17 and 18 **characterized** for acting as a filter and being transparent.

20. Element in accordance with claims 15, 16, 17, 18 and 19 whose application is carried out on mask type protective glasses.

21. Element in accordance with claims 15, 16, 17, 18 and 19 whose application is carried out on shield type protective glasses.
